(19) Europäisches Patentamt
European Patent Office
Office européen des brevets

(11) Veröffentlichungsnummer: **0 628 555 A1**

# (12) EUROPÄISCHE PATENTANMELDUNG

(21) Anmeldenummer: **94108130.9**

(22) Anmeldetag: **26.05.94**

(51) Int. Cl.5: **C07D 401/04**, A61K 31/445, C07D 401/14, C07D 417/14

(30) Priorität: **08.06.93 DE 4319039**

(43) Veröffentlichungstag der Anmeldung: **14.12.94 Patentblatt 94/50**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Bender, Wolfgang, Dr.**
**Kaulbachstrasse 12**
**D-42113 Wupppertal (DE)**
Erfinder: **Häbich, Dieter, Dr.**
**Krummacherstrasse 82**
**D-42115 Wuppertal (DE)**
Erfinder: **Raddatz, Siegfried, Dr.**
**Jakob-Böhme-Strasse 21**
**D-51065 Köln (DE)**
Erfinder: **Röben, Wolfgang, Dr.**
**Strässchen Siefen 30**
**D-51467 Bergisch Gladbach (DE)**
Erfinder: **Wild, Hanno, Dr.**
**Am Ausblick 128**
**D-42113 Wuppertal (DE)**
Erfinder: **Hansen, Jutta, Dr.**
**Pahlkestrasse 98**
**D-42115 Wuppertal (DE)**
Erfinder: **Paessens, Arnold, Dr.**
**Stresemannstrasse 51**
**D-42781 Haan (DE)**

(54) **Substituierte(2-Oxo-1-benzimidazolinyl)-piperidine, Verfahren zu ihrer Herstellung und Verwendung als anti-retrovirale Mittel.**

(57) Die vorliegende Erfindung betrifft substituierte (2-Oxo-1-benzimidazolinyl)-piperidine der allgemeinen Formel (I)

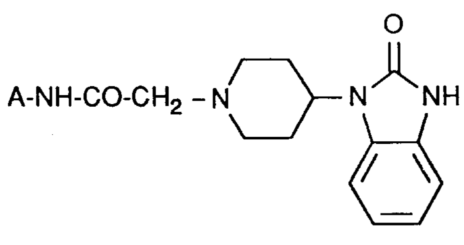

(I)

in welcher
die Substituenten die in der Beschreibung angegebene Bedeutung haben, Verfahren zur ihrer Herstellung und ihre Verwendung als anti-retrovirale Mittel.

EP 0 628 555 A1

Die vorliegende Erfindung betrifft substituierte (2-Oxo-1-benzimidazolinyl)-piperidine der allgemeinen Formel (I)

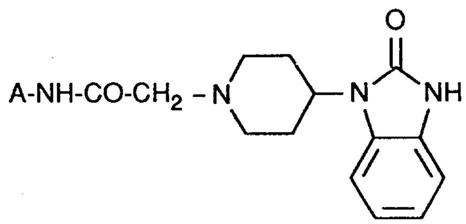

(I)

in welcher

| | |
|---|---|
| A | für Aryl mit 6 bis 10 Kohlenstoffatomen steht, das gegebenenfalls bis zu 4-fach gleich oder verschieden durch Halogen, Carboxy, Nitro, Hydroxy, Trifluormethyl, Cyano, geradkettiges oder verzweigtes Alkyl, Alkoxy, Alkoxycarbonyl oder Alkylthio mit jeweils bis zu 8 Kohlenstoffatomen, durch Cycloalkyl mit 3 bis 7 Kohlenstoffatomen oder durch eine Gruppe der Formel $-P(O)-(OR^1)(OR^2)$, $-CO-R^3$, $-CO-NR^4R^5$, $-SO_2R^6$, $-NR^7R^8$, $-SO_2NR^9R^{10}$, $-D-R^{11}$ oder $-N=NR^{12}$ substituiert ist, worin |
| $R^1$ und $R^2$ | gleich oder verschieden sind und Wasserstoff oder geradkettiges oder verzweigtes Alkyl mit bis zu 6 Kohlenstoffatomen bedeuten, |
| $R^3$ | geradkettiges oder verzweigtes Alkyl mit bis zu 6 Kohlenstoffatomen, Morpholin oder Phenyl bedeutet, das gegebenenfalls durch Halogen substituiert ist, |
| $R^4$ und $R^5$ | gleich oder verschieden sind und Wasserstoff, Cycloalkyl mit 3 bis 7 Kohlenstoffatomen, oder geradkettiges oder verzweigtes Alkyl mit bis zu 6 Kohlenstoffatomen bedeuten, das gegebenenfalls durch Phenyl, Carboxy, Pyridyl oder durch geradkettiges oder verzweigtes Alkoxycarbonyl mit bis zu 6 Kohlenstoffatomen substituiert ist, oder |
| $R^4$ und $R^5$ | gemeinsam mit dem Stickstoffatom einen 5- bis 7-gliedrigen, gesättigten Heterocyclus mit bis zu 2 weiteren Heteroatomen aus der Reihe S, N oder O bilden, der im Fall, daß ein weiteres Stickstoffatom im Ring vorliegt, gegebenenfalls auch über dieses, bis zu 3-fach gleich oder verschieden durch Benzyl, Formyl, Carboxy, geradkettiges oder verzweigtes Alkoxycarbonyl mit bis zu 5 Kohlenstoffatomen, Benzyloxycarbonyl oder durch einen Rest der Formel $-CH_2-CO-NR^{13}R^{14}$ substituiert ist, worin |
| $R^{13}$ und $R^{14}$ | gemeinsam mit dem Stickstoffatom einen 5- bis 7-gliedrigen Heterocyclus bilden, |
| $R^6$ | Phenyl oder geradkettiges oder verzweigtes Alkyl mit bis zu 6 Kohlenstoffatomen bedeutet, |
| $R^7$ und $R^8$ | gleich oder verschieden sind und Wasserstoff, Phenyl, geradkettiges oder verzweigtes Alkyl oder Acyl mit jeweils bis zu 6 Kohlenstoffatomen oder einen Rest der Formel $-C-(S)-NR^{15}R^{16}$ bedeuten, worin |
| $R^{15}$ | Wasserstoff oder Methyl bedeutet und |
| $R^{16}$ | geradkettiges oder verzweigtes Alkoxycarbonyl mit bis zu 6 Kohlenstoffatomen bedeutet, |
| $R^9$ und $R^{10}$ | gleich oder verschieden sind und Wasserstoff, geradkettiges oder verzweigtes Alkyl mit bis zu 6 Kohlenstoffatomen, Phenyl oder einen 5- bis 6-gliederigen, gesättigten oder ungesättigten Heterocyclus mit bis zu 3 weiteren Heteroatomen aus der Reihe S, N oder O bedeuten, wobei die Cyclen gegebenenfalls durch Hydroxy, Carboxy oder durch geradkettiges oder verzweigtes Alkyl, Alkoxy oder Alkoxycarbonyl mit jeweils bis zu 6 Kohlenstoffatomen substituiert sind, |
| D | ein Sauerstoff- oder Schwefelatom bedeutet, |
| $R^{11}$ und $R^{12}$ | gleich oder verschieden sind und Phenyl bedeuten, das gegebenenfalls durch Halogen substituiert ist |

und deren Salze, Verfahren zu ihrer Herstellung und ihre Verwendung als anti-retrovirale Mittel.

Physiologisch unbedenkliche Salze der substituierten (2-Oxo-1-benzimidazolinyl)piperidine können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methylpiperidin.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Heterocyclus steht im allgemeinen für einen 5- bis 7-gliedrigen, bevorzugt 5- bis 6-gliedrigen, gesättigten oder ungesättigten Ring, der als Heteroatome bis zu 3 Sauerstoff-, Schwefel- und/oder Stickstoffatome enthalten kann. Bevorzugt sind 5- und 6-gliedrige Ringe mit einem Sauerstoff-, Schwefel und/oder bis zu 2 Stickstoffatomen. Bevorzugt werden genannt: Thienyl, Furyl, 1,2,4-Thiadiazolyl, Pyrrolyl, Pyrazolyl, Pyridyl, Pyrimidyl, Pyrazinyl, Pyridazinyl, Pyrrolidinyl, Thiazolyl, Oxazolyl, Imidazolyl, Pyrrolidinyl, Piperidinyl, Piperazinyl, Tetrazolyl, Morpholinyl oder Thiomorpholinyl.

Bevorzugt sind Verbindungen der allgemeinen Formel (I),
in welcher

A für Phenyl oder Naphthyl steht, die gegebenenfalls bis zu 4-fach gleich oder verschieden durch Fluor, Chlor, Brom, Carboxy, Nitro, Hydroxy, Trifluormethyl, Cyano, geradkettiges oder verzweigtes Alkyl, Alkoxy, Alkoxycarbonyl oder Alkylthio mit jeweils bis zu 7 Kohlenstoffatomen, durch Cyclopropyl, Cyclopentyl, Cyclohexyl oder durch eine Gruppe der Formel $-P(O)-(OR^1)(OR^2)$, $-CO-R^3$, $-CO-NR^4R^5$, $-SO_2R^6$, $-NR^7R^8$, $-SO_2NR^9R^{10}$, $-D-R^{11}$ oder $-N=N-R^{12}$ substituiert sind,
worin

$R^1$ und $R^2$ gleich oder verschieden sind und Wasserstoff oder geradkettiges oder verzweigtes Alkyl mit bis zu 5 Kohlenstoffatomen bedeuten,

$R^3$ geradkettiges oder verzweigtes Alkyl mit bis zu 5 Kohlenstoffatomen, Morpholin oder Phenyl bedeutet, das gegebenenfalls durch Fluor, Chlor oder Brom substituiert ist,

$R^4$ und $R^5$ gleich oder verschieden sind und Wasserstoff, Cyclopropyl, Cyclopentyl, Cyclohexyl oder geradkettiges oder verzweigtes Alkyl mit bis zu 5 Kohlenstoffatomen bedeuten, das gegebenenfalls durch Phenyl, Carboxy, Pyridyl oder durch geradkettiges oder verzweigtes Alkoxycarbonyl mit bis zu 5 Kohlenstoffatomen substituiert ist,
oder

$R^4$ und $R^5$ gemeinsam mit dem Stickstoffatom einen Piperazin-, Morpholin- oder Piperidinring bilden, die gegebenenfalls im Fall des Piperazinrings, gegebenenfalls auch über das Stickstoffatom, bis zu 2-fach gleich oder verschieden durch Benzyl, Formyl, Carboxy, geradkettiges oder verzweigtes Alkoxycarbonyl mit bis zu 4 Kohlenstoffatomen, Benzyloxycarbonyl oder durch einen Rest der Formel $-CH_2-CO-NR^{13}R^{14}$ substituiert ist,
worin

$R^{13}$ und $R^{14}$ gemeinsam mit dem Stickstoffatom einen Piperazin-, Piperidin- oder Pyrrolidinring bilden,

$R^6$ Phenyl oder geradkettiges oder verzweigtes Alkyl mit bis zu 5 Kohlenstoffatomen bedeutet,

$R^7$ und $R^8$ gleich oder verschieden sind und Wasserstoff, Phenyl, geradkettiges oder verzweigtes Alkyl oder Acyl mit jeweils bis zu 5 Kohlenstoffatomen oder einen Rest der Formel $-C(S)-NR^{15}R^{16}$ bedeuten,
worin

$R^{15}$ Wasserstoff oder Methyl bedeutet
und

$R^{16}$ geradkettiges oder verzweigtes Alkoxycarbonyl mit bis zu 5 Kohlenstoffatomen bedeutet,

$R^9$ und $R^{10}$ gleich oder verschieden sind und Wasserstoff, geradkettiges oder verzweigtes Alkyl mit

bis zu 5 Kohlenstoffatomen, Phenyl oder einen Pyrimidin-, Pyridyl-, Thiazolyl- oder 1,2,4-Thiadiazolylring bedeuten, wobei die Cyclen gegebenenfalls durch Hydroxy, Carboxy oder durch geradkettiges oder verzweigtes Alkyl, Alkoxy oder Alkoxycarbonyl mit jeweils bis zu 5 Kohlenstoffatomen substituiert sind,

D ein Sauerstoff- oder Schwefelatom bedeutet.

$R^{11}$ und $R^{12}$ gleich oder verschieden sind und Phenyl bedeuten, das gegebenenfalls durch Fluor, Chlor oder Brom substituiert ist

und deren Salze.

Besonders bevorzugt sind Verbindungen der allgemeinen Formel (I), in welcher

A für Phenyl oder Naphthyl steht, die gegebenenfalls bis zu 4-fach gleich oder verschieden durch Fluor, Chlor, Brom, Jod, Carboxy, Nitro, Hydroxy, Trifluormethyl, Cyano, geradkettiges oder verzweigtes Alkyl, Alkoxy, Alkoxycarbonyl oder Alkylthio mit jeweils bis zu 6 Kohlenstoffatomen, durch Cyclopropyl, Cyclopentyl, Cyclohexyl oder durch eine Gruppe der Formel $-P(O)-(OR^1)(OR^2)$, $-CO-R^3$, $-CO-NR^4R^5$, $-SO_2R^6$, $-NR^7R^8$, $-SO_2NR^9R^{10}$, $-D-R^{11}$ oder $-N=N-R^{12}$ substituiert sind,

worin

$R^1$ und $R^2$ gleich oder verschieden sind und Wasserstoff oder geradkettiges oder verzweigtes Alkyl mit bis zu 4 Kohlenstoffatomen bedeuten,

$R^3$ geradkettiges oder verzweigtes Alkyl mit bis zu 4 Kohlenstoffatomen, Morpholin oder Phenyl bedeutet, das gegebenenfalls durch Fluor, Chlor oder Brom substituiert ist,

$R^4$ und $R^5$ gleich oder verschieden sind und Wasserstoff, Phenyl, Cyclopropyl, Cyclopentyl, Cyclohexyl oder geradkettiges oder verzweigtes Alkyl mit bis zu 5 Kohlenstoffatomen bedeuten, das gegebenenfalls durch Phenyl, Carboxy, Pyridyl oder durch geradkettiges oder verzweigtes Alkoxycarbonyl mit bis zu 4 Kohlenstoffatomen substituiert ist,

oder

$R^4$ und $R^5$ gemeinsam mit dem Stickstoffatom einen Piperazin-, Morpholin- oder Piperidinring bilden, die gegebenenfalls, im Fall des Piperazinrings, gegebenenfalls auch über das Stickstoffatom, bis zu 2-fach gleich oder verschieden durch Benzyl, Formyl, Carboxy, geradkettiges oder verzweigtes Alkoxycarbonyl mit bis zu 3 Kohlenstoffatomen, Benzyloxycarbonyl oder durch einen Rest der Formel $-CH_2-CO-NR^{13}R^{14}$ substituiert ist,

worin

$R^{13}$ und $R^{14}$ gemeinsam mit dem Stickstoffatom einen Piperazin-, Piperidin- oder Pyrrolidinring bilden,

$R^6$ Phenyl oder geradkettiges oder verzweigtes Alkyl mit bis zu 4 Kohlenstoffatomen bedeutet,

$R^7$ und $R^8$ gleich oder verschieden sind und Wasserstoff, Phenyl, geradkettiges oder verzweigtes Alkyl oder Acyl mit jeweils bis zu 4 Kohlenstoffatomen oder einen Rest der Formel $-C(S)-NR^{15}R^{16}$ bedeuten,

worin

$R^{15}$ Wasserstoff oder Methyl bedeutet

und

$R^{16}$ geradkettiges oder verzweigtes Alkoxycarbonyl mit bis zu 4 Kohlenstoffatomen bedeutet.

$R^9$ und $R^{10}$ gleich oder verschieden sind und Wasserstoff, geradkettiges oder verzweigtes Alkyl mit bis zu 4 Kohlenstoffatomen, Phenyl oder einen Pyrimidin-, Pyridyl-, Thiazolyl- oder 1,2,4-Thiadiazolylring bedeuten, wobei die Cyclen gegebenenfalls durch Hydroxy, Carboxy oder durch geradkettiges oder verzweigtes Alkyl, Alkoxy oder Alkoxycarbonyl mit jeweils bis zu 4 Kohlenstoffatomen substituiert sind,

D ein Sauerstoff- oder Schwefelatom bedeutet,

$R^{11}$ und $R^{12}$ gleich oder verschieden sind und Phenyl bedeuten, das gegebenenfalls durch Fluor, Chlor oder Brom substituiert ist

und deren Salze.

Außerdem wurde ein Verfahren zur Herstellung der erfindungsgemäßen Verbindungen der allgemeinen Formel (I) gefunden, dadurch gekennzeichnet, daß man Verbindungen der allgemeinen Formel (II)

$A-NH-CO-CH_2-L$ (II)

in welcher

A die oben angegebene Bedeutung hat

und

L für eine typische Abgangsgruppe, vorzugsweise für Chlor oder Brom steht,

mit 4-(2-Oxo-1-benzimidazolinyl)-piperidin der Formel (III)

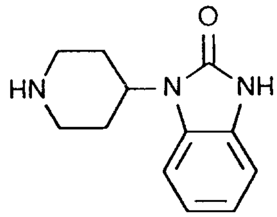

(III)

in inerten Lösemitteln, gegebenenfalls in Anwesenheit einer Base und/oder Hilfsstoffes umsetzt, und gegebenenfalls die unter A aufgeführten Substituenten nach üblichen Methoden, wie beispielsweise Alkylierung, Acyclierung, Verseifung, Hydrierung, Amidierung oder Sulfonamidierung derivatisiert.

Das erfindungsgemäße Verfahren kann durch folgendes Formelschema beispielhaft erläutert werden:

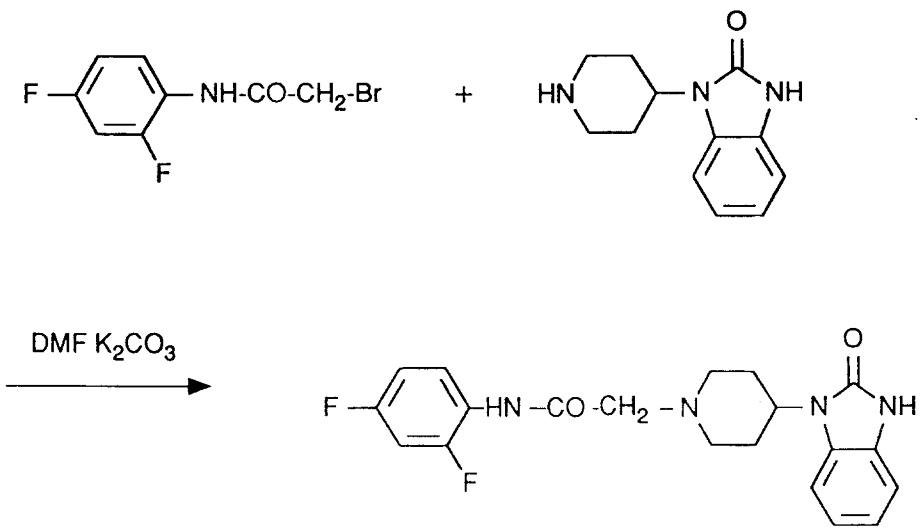


Als Lösemittel eignen sich die üblichen inerten Lösemittel, die sich unter den Reaktionsbedingungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt ist Dichlormethan, Chloroform, Dimethylformamid oder Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Lithiumhydroxid, Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calcium- oder Caesiumcarbonat, oder Alkali- oder Erdalkalialkoholate oder -amide wie Natrium- oder Kaliummethanolat, Natrium- oder Kaliumethanolat oder Kalium-tert.butylat, oder Lithiumdiisopropylamid (LDA), oder organische Amine (Trialkyl($C_1$-$C_6$)-amine) wie Triethylamin, oder Heterocyclen wie 1,4-Diazabicyclo[2.2.2]octan (DABCO), 1,8-Diazabicyclo-[5.4.0]undec-7-en (DBU), Pyridin, Diaminopyridin, N-Methylpiperidin oder Morpholin. Es ist auch möglich als Basen Alkalimetalle, wie Natrium oder deren Hydride wie Natriumhydrid einzusetzen. Bevorzugt sind Triethylamin oder N-Methylmorpholin.

Im allgemeinen setzt man die Base in einer Menge von 0,05 mol bis 10 mol, bevorzugt von 1 mol bis 2 mol, bezogen auf 1 mol der Verbindungen der Formel (III) ein.

Das erfindungsgemäße Verfahren wird im allgemeinen in einem Temperaturbereich von -100 °C bis +100 °C, bevorzugt von 0 °C bis 80 °C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0.5 bis 5 bar).

Als Hilfsstoffe werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Diisopropyl-, N,N'-Dicyclohexylcarbodiimid, N-(3-Dimethylaminoisopropyl)-N'-ethyl-carbodiimid-Hydrochlorid, N-Cyclohexyl-N'-(2-morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxytris(dimethylamino)phosphonium-hexafluorophosphat oder 1-Hydroxybenzotriazol.

Außerdem können beispielsweise Alkalicarbonate, z.B. Natrium- oder Kaliumcarbonat oder -hydrogencarbonat, oder organische Basen wie Trialkylamine, z.B. Triethylamin, Ethyldiisopropylamin, N-Ethylmorpholin, N-Methylpiperidin oder N-Methylmorpholin eingesetzt werden. Bevorzugt ist N-Methylmorpholin.

Die Hilfsstoffe werden in einer Menge von 1,0 Mol bis 3,0 Mol, bevorzugt 1,0 bis 1,2 Mol, bezogen auf jeweils 1 Mol der Verbindungen der allgemeinen Formel (III) eingesetzt.

Die Reaktionen werden in einem Temperaturbereich von -30 °C bis 100 °C, vorzugsweise bei 0 °C bis 30 °C und bei Normaldruck, durchgeführt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von -20 °C bis +100 °C, bevorzugt von 0 °C bis +80 °C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan, Tetrahydrofuran oder Dichlormethan.

Die Abspaltung der Aminoschutzgruppen kann ebenfalls nach üblicher Methode mit Säuren, wie beispielsweise Chlorwasserstoffsäure oder Trifluoressigsäure erfolgen.

Die Abspaltung der Aminoschutzgruppen erfolgt in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen, z.B. Methanol, Ethanol oder Isopropanol.

Die Hydrierung erfolgt im allgemeinen in einem Temperaturbereich von 0 °C bis 80 °C, vorzugsweise von 0 °C bis 40 °C.

Im allgemeinen wird die Hydrierung bei erhöhtem Druck von 2 bar bis 8 bar, vorzugsweise von 3 bis 5 bar, durchgeführt.

Ebenso ist es möglich, insbesondere im Fall von Acylaminoschutzgruppen, diese in einem der oben angegebenen Ethern, mit Hydriden, wie beispielsweise Lithiumaluminiumhydrid oder Natriumborhydrid, bei Raumtemperatur und Normaldruck abzuspalten.

Die Abspaltung der Benzylschutzgruppen erfolgt vorzugsweise mit Ammoniumformiat/Palladium/C in Ethanol/Wasser in einem Temperaturbereich von 50 °C bis 100 °C, vorzugsweise bei 80 °C und Normaldruck.

Die Alkylierung von Aminogruppen erfolgt entweder mit Sulfonsäureestern oder substituierten oder unsubstituierten ($C_1$-$C_8$)-Dialkyl- oder ($C_1$-$C_8$)-Diarylsulfonate, vorzugsweise Methyliodid oder Dimethylsulfat, oder mit Formaldehyd/Natriumborhydrid in einem der oben aufgeführten Ether, vorzugsweise Tetrahydrofuran, in Anwesenheit von Säuren.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C

und Normaldruck.

Die Reduktionen der Carbonylfunkionen der Verbindungen der allgemeinen Formeln (IV) und (VII) sowie partiell auftretender Zwischenprodukte mit dieser Gruppierung erfolgten im allgemeinen mit Reduktionsmitteln, wie beispielsweise Lithiumaluminiumhydrid, mit Boranlösung in Tetrahydrofuran oder mit Boran-Dimethylsulfid (Komplex in Tetrahydrofuran) in einem Temperaturbereich von 0°C bis +70°C, bevorzugt von +20°C bis +65°C und Normaldruck und anschließender Aufnahme in Säuren. Bevorzugt ist Boran-Lösung in Tetrahydrofuran.

Als Säuren für einzelne Verfahrensschritte eignen sich im allgemeinen Protonensäuren wie beispielsweise Salzsäure oder Schwefelsäure. Bevorzugt wird Schwefelsäure eingesetzt.

Die Säure wird im allgemeinen in einer Menge von 1 mol bis 20 mol, bevorzugt von 1 mol bis 5 mol, jeweils bezogen auf 1 mol des Reaktanden, eingesetzt.

Die Umsetzungen werden im allgemeinen in einem Temperaturbereich von -20°C bis +80°C, bevorzugt von 0°C bis +60°C durchgeführt.

Im allgemeinen wird die Umsetzung bei Normaldruck durchgeführt. Es ist aber auch möglich, bei Unterdruck oder bei Überdruck zu arbeiten (z.B. von 0,5 bis 5 bar).

Die Verbindung der allgemeinen Formel (III) ist bekannt.

Die Verbindungen der allgemeinen Formel (II) sind teilweise bekannt oder neu und können dann beispielsweise hergestellt werden, indem man Verbindungen der allgemeinen Formel (IV)

$A\text{-}NH_2$ (IV)

in welcher

A die oben angegebene Bedeutung hat,

mit Verbindungen der allgemeinen Formel (V)

$T\text{-}CO\text{-}CH_2\text{-}L$ (V)

in welcher

L die oben angegebene Bedeutung hat

und

T für Halogen, vorzugsweise für Chlor steht,

in einem der oben aufgeführten Lösemittel und Basen, vorzugsweise Acetonitril und Kaliumcarbonat in einem Temperaturbereich von 0°C bis +80°C, vorzugsweise von +15°C bis +30°C und Normaldruck umsetzt.

Die Verbindungen der allgemeinen Formeln (IV) und (V) sind bekannt.

Die hier beschriebenen Inhibitoren sind Inhibitoren der HIV-Protease und sind als solche für alle Zwecke einsetzbar, für die Enzyminhibitoren geeignet sind. Dies ist zum Beispiel der Einsatz in der Diagnostik, um die Präzision und Selektivität von Enzymaktivitätsmessungen zu verbessern. Bei der Affinitätschromatographie können sie als Affinitätslabel dienen und in der Forschung kann man sie zur Aufklärung von Reaktionsmechanismen und der Spezifität enzymatischer Reaktionen verwenden.

Darüber hinaus wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol. 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50%ige Hemmung der Protease-Aktivität bewirkt.

HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert.

Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Alternativ wurden HIV-suszeptible H9-Zellen anstelle von normalen menschlichen Blutlymphozyten zur Testung der antiviralen Effekte der erfindungsgemäßen Verbindungen eingesetzt.

Die Virusadsorptionslösung wurde zentrifugiert und das infizierte Zellpellet in Wachstumsmedium aufgenommen, so daß 1 x $10^5$ Zellen pro ml eingestellt waren. Die derart infizierten Zellen wurden zu 1 x $10^4$ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten $2^{10}$fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultieren unter diesen Testbedingungen etwa 20 - 50 Syncytien, währen die unbehandelte Zellkontrolle keine Syncytien aufwies.

Die $IC_{50}$-Werte wurden als die Konzentration der behandelten und infizierten Zellen ermittelt, bei der 50% (ca. 10 - 20 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung und Prophylaxe von menschlichen Retrovirusinfektionen.

2.) Für die Behandlung oder Prophylaxe von durch HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV-I oder HTLV-II Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedi-visna (bei Schafen und Ziegen)
b) progressivem Pneumonievirus (PPV) (bei Schafen und Ziegen)
c) caprine arthritis encephalitis Virus (bei Schafen und Ziegen)
d) Zwoegersiekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz (FIV)
h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

**Erklärungen zum experimentellen Teil:**

DC-Systeme:

Stationäre Phase:

Merck DC-Fertigplatten Kieselgel 60 F-254, 5 x 10 cm,, Schichtdicke 0,25 mm, Art.-Nr. 5719

Mobile Phasen (im Test als "DC-System")

I : Toluol/Essigsäureethylester 1:1
II : Essigsäureethylester/Aceton 3:1
III : $CH_2Cl_2$/Methanol 9:1
IV : $NH_3$/$CH_2Cl_2$/Methanol 0,2:9:1
V : HOAc/$CH_2Cl_2$/Methanol 0,2:9:1
VI : Eisessig/n-Butanol/$H_2O$ 1:3:1

HPLC-Systeme:

HPLC-System I:

Säule Merck Lichrosorb$^R$ RP-18, 250-4, 10 µM, Kat.-Nr. 50334

Eluens bei System I

| | |
|---|---|
| A: | pH 7,00 Phosphatpuffer, Merck Art.-Nr. 9439/$H_2O$ 1:50 |
| B: | Acetonitril |
| A/B wi/w 1/1, Fluß: | 2 ml/min, isokratisch, |
| Detektion: | 214 nm |

HPLC-System II:

| | |
|---|---|
| Säule:<br>Eluent: | Nucleosil 120-5 C18, 5 µM, 125 x 4 mm<br>A = 0.01 m $H_3PO_4$, B = Acetonitril |
| Eluentenprogramm: | 0-1 Min.: 10%B<br>1-9 Min.: Gradient mit 10% B/Min.<br>9-13 Min.: 90% B |
| Fluss:<br>Temperatur:<br>Injektionsvolumen:<br>Probe:<br>Detektion: | 2 ml/Min<br>Raumtemperatur<br>5 µl<br>ca. 1 mg/ml<br>UV-Diodenaray bei 210 nm |

Verzeichnis der benutzten Abkürzungen

Allgemeine analytische Methoden

| | |
|---|---|
| DC | Dünnschichtchromatographie |
| GC | Gaschromatograpie |
| HPLC | Hochdruckflüssigkeitschromatographie |
| SC | Säulenchromatographie |
| NMR | Kernspinresonanzspektroskopie (Protonen) |
| MS | Massenspektrometrie (Elektronenstoßionisation) |
| (+) FAB-MS | Fast-atomic-bombardement-Massenspektrometrie, positive Ionen, Matrixsubstanz: m-Nitrobenzylalkohol |
| MS-DCI | Massenspektrometrie, chemische Ionisation |

Reagentien

| | |
|---|---|
| NEM | N-Ethylmorpholin |
| NMM | N-Methylmorpholin |
| TEA | Triethylamin |
| TFA | Trifluoressigsäure |

Lösemittel

| | |
|---|---|
| HOAc | Essigsäure |
| DMF | Dimethylformamid |
| EtOAc | Essigsäureethylester |
| MeOH | Methanol |
| EtOH | Ethanol |
| THF | Tetrahydrofuran |
| DMSO | Dimethylsulfoxid |
| HMPT | Hexamethylphosphorsäuretriamid |
| $CH_2Cl_2$ | Dichlormethan |
| $NH_3$ | Ammoniaklösung (25% wäßrig) |

Schutzgruppen

| | |
|---|---|
| Boc | tert.Butoxycarbonyl |
| Z | Benzyloxycarbonyl |
| DNP | Dinitrophenyl |
| Fmoc | 9-Fluorenylmethoxycarbonyl |
| OEt | Ethylester |
| OMe | Methylester |

Ausgangsverbindungen

Beispiel I

1-Brom-2-(2,4-Difluorphenyl)amino-2-oxoethan

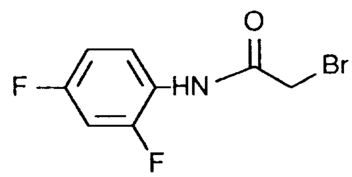

5,16 g (40 mmol) 2,4-Difluoranilin und 6,3 g (40 mmol) Bromessigsäurechlorid werden in 200 ml Dichlormethan mit 5,17 g (40 mmol) Hünig-Base versetzt. Nach 4 h wird 2 mal mit 1 N Salzsäure und 1 mal mit Wasser gewaschen, getrocknet ($MgSO_4$) und eingeengt.

Ausbeute: 7,6g (70%)

$^1$H-NMR ($CDCl_3$): $\delta$ = 4,22 (s, 2H); 6,92 (m, 2H); 8,20 (m, 1H); 8,40 (br, NH).

Beispiel II

1-Chlor-2-(2-carboxy-4-chlorphenyl)amino-2-oxoethan

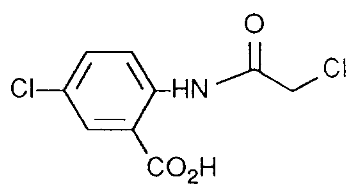

12,5 g (73 mmol) 2-Amino-5-chlorbenzoesäure werden in 500 ml Acetonitril vorgelegt und unter Rühren mit 9,0 g (80 mmol) Chloracetylchlorid versetzt, wobei die Temperatur bei 20-25 °C gehalten wird (Eisbad). Sodann trägt man 22 g (0,16 mol) feinpulverisiertes Kaliumcarbonat ein und rührt die Mischung über Nacht bei Raumtemperatur. Der Ansatz wird mit verdünnter Salzsäure auf pH 2 gestellt und das Acetonitril am Rotationsverdampfer abgezogen. Der Niederschlag wird abgesaugt und gut mit Wasser nachgewaschen. Das Rohprodukt wird mit Natriumbicarbonatlösung versetzt und verrührt Man saugt vom Ungelösten ab und säuert das Filtrat mit verdünnter Salzsäure auf pH 2 an. Das ausgefällte Produkt wird abgesaugt und getrocknet.

Ausbeute: 7 g (77,3% der Theorie)

Fp.: 188 °C

Herstellungsbeispiele

Beispiel I

1-[2-(2,4-Difluorphenyl)amino-2-oxo-ethyl]-4-(2-oxo-1-benzimidazolinyl)piperidin

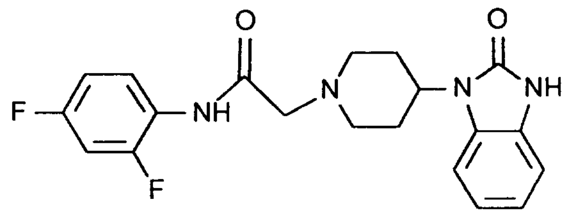

4 g (16 mmol) des Bromessigsäureanilides aus Beispiel I und 4,2 g (19 mmol) 4-(2-Oxo-1-benzimidazolinyl)piperidin in 50 ml Dimethylformamid werden mit 2,6 g (19 mmol) Kaliumcarbonat 18 h bei RT gerührt. Dann wird von Feststoff abgesaugt, zwischen Essigester und Wasser verteilt und 3 mal mit Essigester extrahiert. Die organischen Phasen werden vereinigt, mit gesättigter Natriumchloridlösung gewaschen, getrocknet ($MgSO_4$) und eingeengt. Der erhaltene Rücktand wird mit Ether verrieben, der Feststoff abfiltriert und dann aus Methanol umkristallisiert.

| | |
|---|---|
| Ausbeute: | 4,8 g (77,6% der Theorie) |
| DC-System I: | $R_f$ = 0,16 |
| HPLC-System I: | $R_t$ = 4,07 min |
| (+) FAB-MS: | m/z = 387 M+H |

Beispiel 2

1-[2-(2-Carboxy-4-chlorphenyl)amino-2-oxoethyl]-4-(2-oxo-1-benzimidazolinyl)piperidin

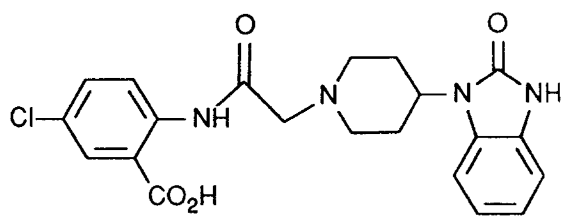

7,94 g (32 mmol) der Verbindung aus Beispiel II und 8,26 g (40 mmol) 4-(2-Oxo-1-benzimidazolinyl)-piperidin (Aldrich) werden in 120 ml DMF vorgelegt. Die Mischung wird mit 5,52 g (40 mmol) Kaliumcarbonat und 0,45 g (3 mmol) Natriumjodid versetzt und über Nacht bei Raumtemperatur gerührt. Der Ansatz wird mit 600 ml $H_2O$ verdünnt und das Produkt durch Zutropfen von 33 ml 1 N Salzsäure bei pH 5 ausgefällt. Der Niederschlag wird abgesaugt, mit 300 ml Diethylether verrührt und im Hochvakuum getrocknet.

| | |
|---|---|
| Ausbeute: | 12,3 g (89,4% der Theorie) |
| DC-System I: | $R_f$ = 0,36 |
| DC-System III: | $R_f$ = 0,25 |
| DC-System V: | $R_f$ = 0,39 |
| HPLC-System I: | $R_t$ = 0,93 min |
| HPLC-System II; | $R_t$ = 4,565 min |
| (+) FAB-MS: | m/z = 429/431 (M+H) |

Beispiel 3

1-[2-(2-Aminophenyl)amino-2-oxoethyl]-4-(2-oxo-1-benzimidazolinyl)piperidin

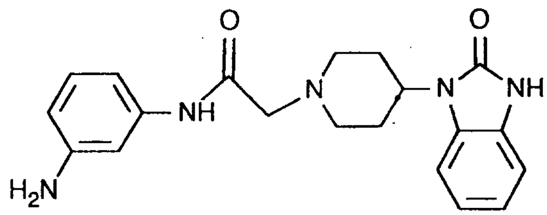

4 g (10,12 mmol) 1-[2-(2-Nitrophenyl)amino-2-oxo-ethyl]-4-(2-oxo-1-benzimidazolinyl)piperidin werden in 40 ml DMF gelöst. Nach Zugabe von 400 mg Pd/C 10% (Aldrich) hydriert man bei 3 bar bis zur vollständigen Abreaktion (DC). Man filtriert vom Katalysator und zeiht alles Lösemittel am Hochvakuum ab. Das Rohprodukt wird mehrmals mit Methanol koevaporiert Die erhaltenen Kristalle werden in Methanol verrührt, abgesaugt und bei 30 ° C im Hochvakuum getrocknet.

| | |
|---|---|
| Ausbeute: | 3,1 g (83,8% der Theorie) |
| DC-System II: | $R_f$ = 0,47 |
| DC-System IV: | $R_f$ = 0,51 |
| HPLC-System I: | $R_t$ = 1,77 min |
| (+) FAB-MS: | m/z 366 (M + H) |

Beispiel 4

1-[2-(4-Amino-2-chlorphenyl)amino-2-oxoethyl]-4-(2-oxo-1-benzimidazolinyl)piperidin

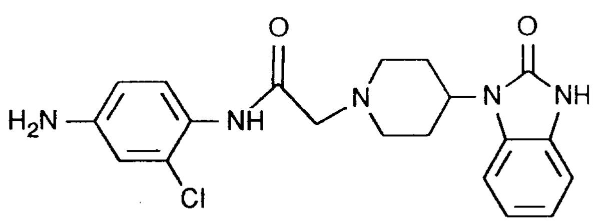

4,29 g (10 mmol) 1-[2-(2-Chlor-5-nitrophenyl)amino-2-oxoethyl]-4-(2-oxo-1-benzimidazolinyl)piperidin, 3,21 g (60 mmol) Ammoniumchlorid, 10 ml Wasser und 60 ml Methylglykol werden zusammengegeben und die Suspension unter Rühren auf 110 ° C erhitzt. Nach Zugabe von 20 ml DMF und 3,35 g (60 mmol) Eisenspäne wird die Mischung 1 h am Rückfluß gekocht. Das Lösemittel wird vollständig im Hochvakuum abgezogen. Der Rückstand wird mit 150 ml Dichlormethan und 100 ml Wasser verrührt und abfiltriert. Das Filtrat wird im Scheidetrichter separiert, und die Wasserphase noch zweimal mit Dichlormethan extrahiert. Die vereinigten organischen Phasen werden mit gesättigter Natriumbicarbonatlösung gewaschen, über Natriumsulfat getrocknet und vom Lösemittel abrotiert. Der erhaltene Feststoff wird mit Diethylether verrührt, abgesaugt und getrocknet.

Ausbeute: 480 mg (12% der Theorie)

Der in Dichlormethan/Wasser unlösliche Feststoff wird mit 150 ml Methanol heiß ausgerührt, abfiltriert und mit 50 ml heißem Methanol nachgewaschen. Die Filtrate werden zur Trockene eingeengt und der Rückstand nach Verreiben mit Diethylether abgesaugt und getrocknet.

| | |
|---|---|
| Ausbeute: | 2,48 g (62% der Theorie) |
| Gesamtausbeute: | 2,96 g (74% der Theorie) |
| DC (9/1): | $R_f$ = 0,43 |
| HPLC: | 2,11 min |

(+) FAB-MS: m/z 400/402 M + H

Die in Tabelle 1 aufgeführten Verbindungen werden analog der Vorschriften der Beispiele 1 - 4 und ausgehend von den entsprechenden Haloacetamidderivaten und 4-(2-Oxo-1-benzimidazolinyl)piperidin (Aldrich, Jansen) hergestellt.

Die verwendeten Halbacetamidderivate werden analog Beispiele I und II aus den entsprechenden Arylaminen und Brom- bzw. Chloracetylchlorid synthetisiert. Einige der Haloacetamidderivate wurden zugekauft (Fa. Bader)

Die eingesetzten Arylamine sind entweder kommerziell erhältlich oder werden nach bekannten Methoden (Houben-Weyl) - gegebenenfalls über die Nitroverbindung als Vorstufe - hergestellt. Die Reduktion der Nitro- zur Aminogruppe erfolgt beispielsweise durch katalytische Hydrierung oder durch Umsetzung mit unedlen Metallen wie Eisen oder Zink.

Tabelle 1:

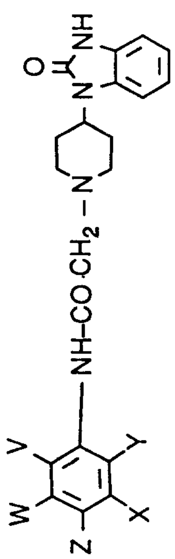

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 5 | H | H | H | Cl | Cl | I/ 0,27 | I/ 12,58 | 419/421 (M+H) |
| 6 | H | $NO_2$ | H | OH | H | II/ 0,60 | I/ 1,76 | 412 (M+H) |
| 7 | $CH_3$ | H | H | $CH_3$ | H | II/ 0,68 | I/ 2,78 | 379 (M+H) |
| 8 | $-CO-C_6H_4$-o-Cl | H | H | H | Cl | II/ 0,83 | I/ 19,16 | 523/525 (M+H) |
| 9 | $CH_3$ | $CH_3$ | H | H | H | II/0,26 | I/3,76 | 379 (M+H) |
| 10 | $CH_3$ | H | H | H | H | II/0,31 | I/3,21 | 365 (M+H) |
| 11 | $CH_3$ | Cl | H | H | H | II/0,50 | I/4,94 | 399/401 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 12 | H | H | H | H | H | II/0,45 | I/2,61 | 351 (M+H) |
| 13 | H | H | H | H | $CH_3O$ | II/0,50 | I/2,32 | 381 (M+H) |
| 14 | $CH_3$ | H | Cl | H | H | II/0,56 | I/4,89 | 399/401 (M+H) |
| 15 | $NO_2$ | H | H | H | H | II/0,43 | I/5,17 | 396 (M+H) |
| 16 | H | $NO_2$ | H | H | H | II/0,67 | I/3,21 | 396 (M+H) |
| 17 | H | $CF_3$ | H | H | H | II/0,65 | I/4,80 | 419 (M+H) |
| 18 | OH | H | H | H | H | II/0,47 | I/2,04 | 367 (M+H) |
| 19 | $H_3C-CH_2-CH-CH_3$ | | H | H | H | II/0,43 | I/6,30 | 407 (M+H) |
| 20 | $-CH(CH_3)_2$ | H | H | H | H | II/0,52 | I/4,93 | 393 (M+H) |
| 21 | $-O-C_6H_5$ | H | H | H | H | II/0,53 | I/8,22 | 443 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 22 | 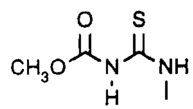 | H | H | H | $-S-C_6H_5$ | II/0,62 | I/8,31 | 591 (M+H) |
| 23 | $NO_2$ | H | H | H | $-SO_2-C_6H_5$ | II/0,41 | I/6,77 | 536 (M+H) |
| 24 | H | Cl | H | H | H | II/0,64 | I/4,26 | 385/387 (M+H) |
| 25 | H | Cl | H | H | Cl | II/0,45 | I/6,46 | 419/421 (M+H) |
| 26 | H | NC | H | H | NC- | II/0,37 | I/2,77 | 401 (M+H) |
| 27 | H | H | H | H | $-CO_2-CH_3$ | II/0,58 | I/2,84 | 409 (M+H) |
| 28 | H | H | H | H | $-CH_3$ | II/0,42 | I/3,56 | 365 (M+H) |
| 29 | H | H | H | H | Cl | II/0,66 | I/2,31 | 385/387 (M+H) |
| 30 | H | Cl | H | H | $NO_2$ | II/0,66 | I/4,34 | 430/432 (M+H) |
| 31 | H | H | H | H | $CF_3$ | II/0,67 | I/5,22 | 419 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 32 | H | H | H | H | $-C(CH_3)_3$ | II/0,64 | I/8,49 | 407 (M+H) |
| 33 | H | H | H | H | NC- | II/0,65 | I/2,57 | 376 (M+H) |
| 34 | H | H | H | H | $-NO_2$ | II/0,65 | I/3,33 | 396 (M+H) |
| 35 | H | H | H | H | $-CO-CH_3$ | II/0,61 | I/2,22 | 393 (M+H) |
| 36 | H | H | H | H | $-N-(CH_3)_2$ | II/0,39 | I/2,96 | 394 (M+H) |
| 37 | H | H | H | H | $-O-C_6H_5$ | II/0,44 | I/6,91 | 443 (M+H) |
| 38 | H | H | H | H | $-C_6H_{11}$ | II/0,54 | I/17,83 | 433 (M+H) |
| 39 | H | H | H | H | $-NH-CO-CH_3$ | II/0,14 | I/1,57 | 408 (M+H) |
| 40 | H | H | H | H | $-CO-NH_2$ | II/0,10 | I/1,43 | 394 (M+H) |
| 41 | H | H | H | H | $-SO_2-NH_2$ | II/0,31 | I/1,59 | 430 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 42 | H | H | H | H | 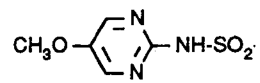 | II/0,30 | I/1,11 | 538 (M+H) |
| 43 | H | H | H | H | $-N=N-C_6H_5$ | II/0,69 | I/12,62 | 455 (M+H) |
| 44 | H | H | H | H | $-CO-C_6H_5$ | II/0,58 | I/4,66 | 455 (M+H) |
| 45 | Cl | H | H | H | H | II/0,69 | I/6,09 | 385/387 (M+H) |
| 46 | $-CH_3$ | $-NO_2$ | H | H | H | II/0,62 | I/3,16 | 410 (M+H) |
| 47 | H | H | H | H | $-OC_2H_5$ | II/0,54 | I/3,35 | 395 (M+H) |
| 48 | H | H | H | H | 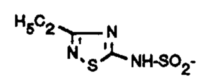 | II/0,13<br>III/0,25 | I/0,93 | 542 (M+H) |
| 49 | $NO_2$ | H | H | H | $-SC_3H_7$ | II/0,63 | I/22,45 | 470 (M+H) |
| 50 | $NO_2$ | H | H | H | Cl | II/0,66 | I/9,70 | 430/432 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 51 | $-C_2H_5$ | H | H | H | H | II/0,63 | I/4,21 | 379 (M+H) |
| 52 | $CH_3$ | H | H | H | Cl | II/0,66 | I/5,36 | 399/401 (M+H) |
| 53 | $-CO-NH_2$ | H | H | H | $NO_2$ | II/0,58 | I/2,65 | 439 (M+H) |
| 54 | $-O-C_4H_9$ | H | H | H | H | II/0,58 | I/9,19 | 423 (M+H) |
| 55 | $-C(CH_3)_3$ | H | H | H | H | II/0,43 | I/5,94 | 407 (M+H) |
| 56 | $CF_3$ | H | H | H | Cl | II/0,67 | I/10,97 | 453/455 (M+H) |
| 57 | $-CO_2H$ | H | H | H | H | II/0,30 | I/0,96 | 395 (M+H) |
| 58 | $-OCH_3$ | H | H | H | Cl | II/0,73 | I/6,86 | 415/417 (M+H) |
| 59 | $-CO_2C_2H_5$ | H | H | H | H | II/0,55 | I/10,51 | 423 (M+H) |
| 60 | $-CO-NH-CH_3$ | H | H | H | H | II/0,62 | I/2,43 | 408 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 61 | $CH_3$ | H | H | H | $-NO_2$ | II/0,60 | I/4,44 | 410 (M+H) |
| 62 | $-CO-NH-CH_2-COOC_2H_5$ | H | H | H | H | II/0,47 | I/2,65 | 480 (M+H) |
| 63 | $-CO-NH_2$ | H | H | H | H | II/0,51 | I/1,96 | 394 (M+H) |
| 64 | $-CO_2H$ | H | Cl | H | H | III/0,14 | II/4,585 | 429/431 (M+H) |
| 65 | $-SO_2-NH-C_6H_4$-o-$COOCH_3$ | | H | H | H | II/0,47 | I/11,48 | 564 (M+H) |
| 66 | Cl | H | H | H | $CF_3$ | II/0,61 | I/11,87 | 453/455 (M+H) |
| 67 | H | H | H | H | HO- | II/0,20 | I/2,14 | 367 (M+H) |
| 68 | Cl | H | H | H | $NO_2$ | III/0,66 | I/8,40 | 430/432 (M+H) |
| 69 | H | H | H | H | Br | II/0,47 | I/4,90 | 429/431 (M+H) |
| 70 | $-CO-C_6H_5$ | H | H | H | Cl | II/0,59 | I/19,50 | 489/391 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 71 | H | H | H | H | 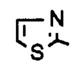 NH-$SO_2$- | II/0,28 | I/1,05 | 513 (M+H) |
| 72 | H | H | H | H | 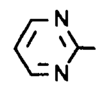 NH-$SO_2$- | II/0,32 | I/0,87 | 508 (M+H) |
| 73 | H | H | H | H | -$NH_2$ | II/0,36 | I/1,74 | 366 (M+H) |
| 74 | H | Cl | H | H | Br | II/0,57 | I/7,57 | 463/465 (M+H) |
| 75 | H | $NO_2$ | H | H | Cl | II/0,64 | I/4,66 | 430/432 (M+H) |
| 76 | $CH_3$ | $NH_2$ | H | H | H | II/0,59 | I/1,96 | 380 (M+H) |
| 77 | $NH_2$ | H | H | H | -S-$C_3H_7$ | II/0,27 | I/5,08 | 440 (M+H) |
| 78 | H | -$N(CH_3)_2$ | H | H | H | II/0,43 | I/3,46 | 394 (M+H) |
| 79 | H | H | H | H | -NH-$C_6H_5$ | II/0,57 | I/5,11 | 442 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 80 | H | $-N(C_2H_5)_2$ | H | H | H | II/0,62 | I/6,87 | 422 (M+H) |
| 81 | $-NH_2$ | H | H | H | $-SO_2C_6H_5$ | II/0,47 | I/2,82 | 506 (M+H) |
| 82 | $-NH_2$ | H | H | H | Cl | III/0,33 | I/3,49 | 400/402 (M+H) |
| 83 | $-CONH_2$ | H | H | H | $-NH_2$ | II/0,29 | I/1,20 | 409 (M+H) |
| 84 | $CH_3$ | H | H | H | $-NH_2$ | III/0,56 | I/1,64 | 380 (M+H) |
| 85 | $-NH_2$ | H | H | H | H | II/0,19 | I/2,06 | 366 (M+H) |
| 86 | -OH | H | $-NH_2$ | H | H | III/0,28 | I/1,09 | 382 (M+H) |
| 87 | Br | H | H | Br | $-C_2H_5$ | II/0,59 | I/7,78 | 535/537/539 (M+H) |
| 88 | Cl | H | H | Br | Br | II/0,50 | I/5,74 | 541/543/545 (M+H) |
| 89 | Br | H | Hr | Br | Cl | I/0,31 | I/6,05 | 541/543/545 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 90 | $NO_2$ | H | H | H | $-OCH_3$ | I/0,33<br>II/0,60 | I/5,78 | 426<br>(M+H) |
| 91 | Cl | H | H | Cl | Cl | I/0,37<br>II/0,69 | I/21,63 | 453/455/457<br>(M+H) |
| 92 | Cl | H | Cl | H | Cl | I/0,13<br>II/0,55 | I/5,52 | 453/455/457<br>(M+H) |
| 93 | $CH_3$ | H | H | $CH_3$ | Br | I/0,25<br>II/0,54 | I/5,48 | 457/459<br>(M+H) |
| 94 | $-O-C_6H_5$ | H | H | H | Cl | I/0,15<br>II/0,51 | I/16,28 | 477/479<br>(M+H) |
| 95 | $-CO_2H$ | H | H | H | J | III/0,29 | | 521<br>(M+H) |
| 96 | $-CO-C_6H_5$ | H | Cl | H | H | I/0,10<br>II/0,38 | I/19,10 | 489/491<br>(M+H) |
| 97 | $-OCH_3$ | H | H | H | $NO_2$ | II/0,62 | I/5,50 | 426<br>(M+H) |
| 98 | $CH_3$ | H | $CH_3$ | H | H | I/0,07<br>II/0,39 | I/4,63 | 379<br>(M+H) |
| 99 | $-COOCH_3$ | H | H | H | Cl | II/0,61 | I/15.40<br>II/5,398 | 443/445<br>(M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 100 | Br | H | H | H | $NO_2$ | II/0,54 | I/8,20 | 474/476 (M+H) |
| 101 | H | Cl | H | H | $-O-C_6H_4-p-Cl$ | I/0,22 II/0,71 | I/17,47 | 511/513 (M+H) |
| 102 | $-CO_2H$ | H | $-NO_2$ | H | H | III/0,09 | | 440 (M+H) |
| 103 | H | F | H | H | H | I/0,06 II/0,61 | I/3,42 | 369 (M+H) |
| 104 | F | H | H | H | H | I/0,09 II/0,54 | I/3,96 | 369 (M+H) |
| 105 | $-COOCH_3$ | H | Cl | H | H | II/0,53 | I/15,40 II/5,498 | 443/445 (M+H) |
| 106 | $NO_2$ | H | H | H | F | I/0,66 II/0,89 | I/6,50 | 414 (M+H) |
| 107 | $-COOC_2H_5$ | H | Cl | H | H | I/0,26 II/0,71 | I/24,16 | 457/459 (M+H) |
| 108 | $-CONH_2$ | F | H | H | H | I/0,07 II/0,57 | I/2,30 | 411 (M+H) |
| 109 | F | H | F | H | H | I/0,14 II/0,60 | I/4,86 | |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 110 | F | F | H | H | H | I/0,07<br>II/0,60 | I/3,98 | 387<br>(M+H) |
| 111 | H | F | H | H | F | I/0,06<br>II/0,65 | I/3,63 | 387<br>(M+H) |
| 112 | F | H | F | F | H | I/0,14<br>II/0,64 | I/2,35 | 387<br>(M+H) |
| 113 | H | H | H | H | F | I/0,14<br>II/0,57 | I/2,98 | 369<br>(M+H) |
| 114 | $-CONH_2$ | H | H | H | Br | II/0,58 | I/3,29 | 472/474<br>(M+H) |
| 115 | $-CONH_2$ | H | H | H | Cl | I/0,12<br>II/0,50 | I/2,99 | 428/430<br>(M+H) |
| 116 | $-CO-NH-(CH_2)_2-C_6H_5$ | H | H | H | Cl | II/0,59 | I/13,86 | 532/534<br>(M+H) |
| 117 | H | $CO_2H$ | H | H | Cl | V/0,08 | II/3,971 | 429/431<br>(M+H) |
| 118 | $-CO-NH-CH_2-C_6H_5$ | H | H | H | Cl | V/0,75 | I/9,48 | 518/520<br>(M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 119 | 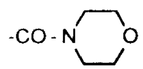 | H | H | H | Cl | V/0,76 | I/3,10<br>II/4,055 | 498/500 (M+H) |
| 120 | Cl | H | H | $CO_2H$ | Cl | V/0,21 | II/4,438 | 463/465 (M+H) |
| 121 | Cl | H | $CO_2H$ | H | H | V/0,40 | II/3,925 | 429/431 (M+H) |
| 122 | 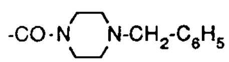 | H | H | H | Cl | IV/0,90 | I/10,18 | 587/589 (M+H) |
| 123 | 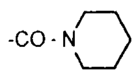 | H | H | H | Cl | IV/0,87 | I/6,75 | 496/498 (M+H) |
| 124 | $-CO-NH-CH_2-CH(CH_3)_2$ | H | H | H | Cl | V/0,90 | I/10,77<br>II/5,237 | 484/486 (M+H) |
| 125 | $-CO_2H$ | H | H | H | Br | V/0,27 | II/4,863 | 473/475 (M+H) |
| 126 | H | F | F | H | H | V/0,51 | I/4,16 | 387 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 127 | 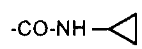 | H | H | H | Cl | V/0,83 | I/6,02 | 468/470 (M+H) |
| 128 | 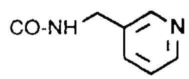 | H | H | H | Cl | V/0,83 | I/4,53 | 519/521 (M+H) |
| 129 | 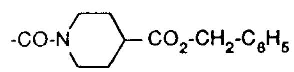 | H | H | H | Cl | V/0,77 | I/12,62 | 630 (M+H) |
| 130 | $NH_2$ | 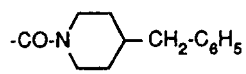 | Cl | H | H | V/0,81 | I/21,48 | 586 (M+H) |
| 131 | $-CO_2H$ | H | H | H | F | V/0,36 | II/4,300 | 413 (m+H) |
| 132 | $-CO_2H$ | H | H | H | $-CH_3$ | V/0,36 | II/4,603 | 409 (M+H) |
| 133 | $-CO_2H$ | H | H | H | $-CF_3$ | V/0,34 | II/5,104 | 463 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 134 | H | Cl | H | H | $-CO_2H$ | V/0,12 | II/3,882 | 429/431 (M+H) |
| 135 | $-CO_2H$ | H | $-OCH_3$ | H | $-OCH_3$ | V/0,36 | I/1,12<br>II/3,871 | 455 (M+H) |
| 136 | $-CO-N(C_4H_9)_2$ | H | H | H | Cl | V/0,81 | I/4,13 | 540/542 (M+H) |
| 137 | $-CO-N(C_2H_5)_2$ | H | H | H | Cl | V/0,78 | I/6,73 | 484/486 (M+H) |
| 138 | $-CO_2H$ | H | H | H | $NO_2$ | V/0,38 | II/4,157 | 440 (M+H) |
| 139 | $-CO_2CH_3$ | H | $-CO_2CH_3$ | H | H | V/0,57 | I/9,82 | 467 (M+H) |
| 140 | H | $-CO_2CH_3$ | $-CO_2CH_3$ | H | H | V/0,41 | I/3,41 | 467 (M+H) |
| 141 | $-CO_2H$ | H | F | H | H | V/0,61 | II/4,475 | 413 (M+H) |
| 142 | $-CO-NH-C_6H_{11}$ | H | H | H | Cl | V/0,79 | I/12,82 | 510/512 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 143 | 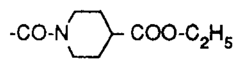 | H | H | H | Cl | V/0,78 | I/9,64 | 568/570 (M+H) |
| 144 | $-CO-NH-CH_2-C(CH_3)_2$ | H | H | H | Cl | V/0,81 | I/11,91 | 498/500 (M+H) |
| 145 | $-COOC_2H_5$ | H | H | H | Cl | V/0,80 | I/21,58 | 457/459 (M+H) |
| 146 | $-COOCH_3$ | H | $OCH_3$ | $OCH_3$ | H | V/0,57 | I/2,55 | 499 (M+H) |
| 147 | $-CO_2H$ | H | $C_2H_5$ | H | H | V/0,53 | II/4,686 | 423 (M+H) |
| 148 | $CO_2H$ | H | H | J | J | V/0,56 | II/5,471 | 647 (M+H) |
| 149 | 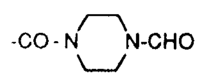 | H | H | H | Cl | V/0,90 | II/3,729 | 525/527 (M+H) |
| 150 | 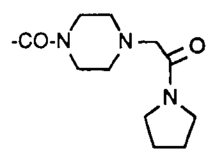 | H | H | H | Cl | V/0,68 | I/2,84 | 608/610 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 151 | $-CO_2H$ | $CO_2H$ | H | H | H | VI/0,34 | II/3,031 | 439 (M+H) |
| 152 | $-PO(OC_2H_5)_2$ | H | H | Cl | Cl | V/0,57 | I/20.08 | 555/557 (M+H) |
| 153 | -CO-NH 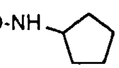 | H | H | H | Cl | V/0,83 | I/11,32 | 496/498 (M+H) |
| 154 | $-CO-NH-CH_3$ | H | H | H | Cl | V/0.76 | I/3,82 II/4,062 | 442/444 (M+H) |
| 155 | $-PO(OH)(OC_2H_5)$ | H | H | H | Cl | V/0.07 | II/4,473 | 527/529 (M+H) |
| 156 | $NH_2$ | H | H | H | F | V/0,41 | | 384 (M+H) |
| 157 | $-CO_2H$ | H | $-CO_2H$ | H | H | V/0,15 | II/3,391 | 439 (M+H) |
| 158 | H | $CO_2H$ | $-CO_2H$ | H | H | VI/0,60 | II/3,329 | 439 (M+H) |
| 159 | $-CO_2H$ | H | $-OCH_3$ | $-OCH_3$ | $-OCH_3$ | VI/0,58 | II/3,894 | 485 (M+H) |

Fortsetzung Tabelle 1:

| Bsp.-Nr. | V | W | X | Y | Z | DC-System/ $R_f$-Wert | HPLC-System/ $R_t$-Wert (min.) | (+) FAB-MS m/z |
|---|---|---|---|---|---|---|---|---|
| 160 | 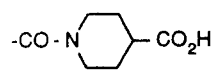 | H | H | H | Cl | VI/0,59 | II/4,310 | 540/542 (M+H) |
| 161 | $-CO_2H$ | H | H | H | $-NH_2$ | V/0,34 | I/0,75 | 410 (M+H) |

Tabelle 2:

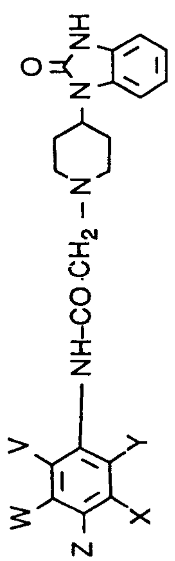

| Bsp.-Nr. | V W | Z | Y | X | DC-System/ $R_f$-Wert | HPLC-System/ $R_t$-Wert (min.) | (+) FAB-MS m/z |
|---|---|---|---|---|---|---|---|
| 162 | 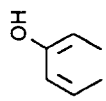 | H | H | H | | I/3,57 | 417 (M+H) |
| 163 | 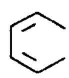 | $-OC_2H_5$ | H | H | II/0,55 | I/6,65 | 445 (M+H) |
| 164 | H | $H_9C_4O$ 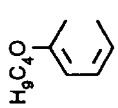 | H | H | II/0,50 | I/18,96 | 473 (M+H) |
| 165 | 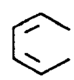 | $OC_4H_9$ | H | H | II/0,57 | I/18,07 | 473 (M+H) |
| 166 | 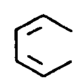 | $OC_3H_7$ | H | H | II/0,41 | I/11,21 | 459 (M+H) |
| 167 | H | 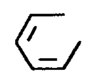 | H | H | V/0,37 | II/5,071 | 445 (M+H) |

## Patentansprüche

1. Substituierte (2-Oxo-1-benzimidazolinyl)-piperidine der allgemeinen Formel (I)

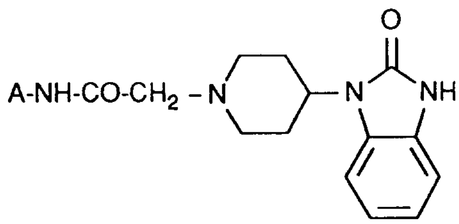

(I)

in welcher

A für Aryl mit 6 bis 10 Kohlenstoffatomen steht, das gegebenenfalls bis zu 4-fach gleich oder verschieden durch Halogen, Carboxy, Nitro, Hydroxy, Trifluormethyl, Cyano, geradkettiges oder verzweigtes Alkyl, Alkoxy, Alkoxycarbonyl oder Alkylthio mit jeweils bis zu 8 Kohlenstoffatomen, durch Cycloalkyl mit 3 bis 7 Kohlenstoffatomen oder durch eine Gruppe der Formel $-P(O)-(OR^1)(OR^2)$, $-CO-R^3$, $-CO-NR^4R^5$, $-SO_2R^6$, $-NR^7R^8$, $-SO_2NR^9R^{10}$, $-D-R^{11}$ oder $-N=NR^{12}$ substituiert ist,

worin

$R^1$ und $R^2$ gleich oder verschieden sind und Wasserstoff oder geradkettiges oder verzweigtes Alkyl mit bis zu 6 Kohlenstoffatomen bedeuten,

$R^3$ geradkettiges oder verzweigtes Alkyl mit bis zu 6 Kohlenstoffatomen, Morpholin oder Phenyl bedeutet, das gegebenenfalls durch Halogen substituiert ist,

$R^4$ und $R^5$ gleich oder verschieden sind und Wasserstoff, Cycloalkyl mit 3 bis 7 Kohlenstoffatomen, oder geradkettiges oder verzweigtes Alkyl mit bis zu 6 Kohlenstoffatomen bedeuten, das gegebenenfalls durch Phenyl, Carboxy, Pyridyl oder durch geradkettiges oder verzweigtes Alkoxycarbonyl mit bis zu 6 Kohlenstoffatomen substituiert ist,

oder

$R^4$ und $R^5$ gemeinsam mit dem Stickstoffatom einen 5- bis 7-gliedrigen, gesättigten Heterocyclus mit bis zu 2 weiteren Heteroatomen aus der Reihe S, N oder O bilden, der im Fall, daß ein weiteres Stickstoffatom im Ring vorliegt, gegebenenfalls auch über dieses, bis zu 3-fach gleich oder verschieden durch Benzyl, Formyl, Carboxy, geradkettiges oder verzweigtes Alkoxycarbonyl mit bis zu 5 Kohlenstoffatomen, Benzyloxycarbonyl oder durch einen Rest der Formel $-CH_2-CO-NR^{13}R^{14}$ substituiert ist,

worin

$R^{13}$ und $R^{14}$ gemeinsam mit dem Stickstoffatom einen 5- bis 7-gliedrigen Heterocyclus bilden,

$R^6$ Phenyl oder geradkettiges oder verzweigtes Alkyl mit bis zu 6 Kohlenstoffatomen bedeutet,

$R^7$ und $R^8$ gleich oder verschieden sind und Wasserstoff, Phenyl, geradkettiges oder verzweigtes Alkyl oder Acyl mit jeweils bis zu 6 Kohlenstoffatomen oder einen Rest der Formel $-C(S)-NR^{15}R^{16}$ bedeuten,

worin

$R^{15}$ Wasserstoff oder Methyl bedeutet

und

$R^{16}$ geradkettiges oder verzweigtes Alkoxycarbonyl mit bis zu 6 Kohlenstoffatomen bedeutet,

$R^9$ und $R^{10}$ gleich oder verschieden sind und Wasserstoff, geradkettiges oder verzweigtes Alkyl mit bis zu 6 Kohlenstoffatomen, Phenyl oder einen 5- bis 6-gliederigen, gesättigten oder ungesättigten Heterocyclus mit bis zu 3 weiteren Heteroatomen aus der Reihe S, N oder O bedeuten, wobei die Cyclen gegebenenfalls durch Hydroxy, Carboxy oder durch geradkettiges oder verzweigtes Alkyl, Alkoxy oder Alkoxycarbonyl mit jeweils bis zu 6 Kohlenstoffatomen substituiert sind,

D ein Sauerstoff- oder Schwefelatom bedeutet,

$R^{11}$ und $R^{12}$ gleich oder verschieden sind und Phenyl bedeuten, das gegebenenfalls durch Halogen substituiert ist

und deren Salze.

2. Verbindungen der allgemeinen Formel (I), gemäß Anspruch 1 in welcher

A für Phenyl oder Naphthyl steht, die gegebenenfalls bis zu 4-fach gleich oder verschieden durch Fluor, Chlor, Brom, Carboxy Nitro, Hydroxy, Trifluormethyl, Cyano, geradkettiges oder verzweigtes Alkyl, Alkoxy, Alkoxycarbonyl oder Alkylthio mit jeweils bis zu 7 Kohlenstoffatomen, durch Cyclopropyl, Cyclopentyl, Cyclohexyl oder durch eine Gruppe der Formel $-P(O)-(OR^1)(OR^2)$, $-CO-R^3$, $-CO-NR^4R^5$, $-SO_2R^6$, $-NR^7R^8$, $-SO_2NR^9R^{10}$, $-D-R^{11}$ oder $N=N-R^{12}$ substituiert sind,

worin

$R^1$ und $R^2$ gleich oder verschieden sind und Wasserstoff oder geradkettiges oder verzweigtes Alkyl mit bis zu 5 Kohlenstoffatomen bedeuten,

$R^3$ geradkettiges oder verzweigtes Alkyl mit bis zu 5 Kohlenstoffatomen, Morpholin oder Phenyl bedeutet, das gegebenenfalls durch Fluor, Chlor oder Brom substituiert ist,

$R^4$ und $R^5$ gleich oder verschieden sind und Wasserstoff, Cyclopropyl, Cyclopentyl, Cyclohexyl oder geradkettiges oder verzweigtes Alkyl mit bis zu 5 Kohlenstoffatomen bedeuten, das gegebenenfalls durch Phenyl, Carboxy, Pyridyl oder durch geradkettiges oder verzweigtes Alkoxycarbonyl mit bis zu 5 Kohlenstoffatomen substituiert ist,

oder

$R^4$ und $R^5$ gemeinsam mit dem Stickstoffatom einen Piperazin-, Morpholin- oder Piperidinring bilden, die gegebenenfalls im Fall des Piperazinrings, gegebenenfalls auch über das Stickstoffatom, bis zu 2-fach gleich oder verschieden durch Benzyl, Formyl, Carboxy, geradkettiges oder verzweigtes Alkoxycarbonyl mit bis zu 4 Kohlenstoffatomen, Benzyloxycarbonyl oder durch einen Rest der Formel $-CH_2-CO-NR^{13}R^{14}$ substituiert ist,

worin

$R^{13}$ und $R^{14}$ gemeinsam mit dem Stickstoffatom einen Piperazin-, Piperidin- oder Pyrrolidinring bilden,

$R^6$ Phenyl oder geradkettiges oder verzweigtes Alkyl mit bis zu 5 Kohlenstoffatomen bedeutet,

$R^7$ und $R^8$ gleich oder verschieden sind und Wasserstoff, Phenyl, geradkettiges oder verzweigtes Alkyl oder Acyl mit jeweils bis zu 5 Kohlenstoffatomen oder einen Rest der Formel $-C(S)-NR^{15}R^{16}$ bedeuten,

worin

$R^{15}$ Wasserstoff oder Methyl bedeutet

und

$R^{16}$ geradkettiges oder verzweigtes Alkoxycarbonyl mit bis zu 5 Kohlenstoffatomen bedeutet,

$R^9$ und $R^{10}$ gleich oder verschieden sind und Wasserstoff, geradkettiges oder verzweigtes Alkyl mit bis zu 5 Kohlenstoffatomen, Phenyl oder einen Pyrimidin-, Pyridyl-, Thiazolyl- oder 1,2,4-Thiadiazolylring bedeuten, wobei die Cyclen gegebenenfalls durch Hydroxy, Carboxy oder durch geradkettiges oder verzweigtes Alkyl, Alkoxy oder Alkoxycarbonyl mit jeweils bis zu 5 Kohlenstoffatomen substituiert sind,

D ein Sauerstoff- oder Schwefelatom bedeutet,

$R^{11}$ und $R^{12}$ gleich oder verschieden sind und Phenyl bedeuten, das gegebenenfalls durch Fluor, Chlor oder Brom substituiert ist

und deren Salze.

3. Verbindungen der allgemeinen Formel (I), gemäß Anspruch 1 in welcher

A für Phenyl oder Naphthyl steht, die gegebenenfalls bis zu 4-fach gleich oder verschieden durch Fluor, Chlor, Brom, Jod, Carboxy, Nitro, Hydroxy, Trifluormethyl, Cyano, geradkettiges oder verzweigtes Alkyl, Alkoxy, Alkoxycarbonyl oder Alkylthio mit jeweils bis zu 6 Kohlenstoffatomen, durch Cyclopropyl, Cyclopentyl, Cyclohexyl oder durch eine Gruppe der Formel $-P(O)-(OR^1)(OR^2)$, $-CO-R^3$, $-CO-NR^4R^5$, $-SO_2R^6$, $-NR^7R^8$, $-SO_2NR^9R^{10}$, $-D-R^{11}$ oder $-N=N-R^{12}$ substituiert sind,

worin

$R^1$ und $R^2$ gleich oder verschieden sind und Wasserstoff oder geradkettiges oder verzweigtes Alkyl mit bis zu 4 Kohlenstoffatomen bedeuten,

$R^3$ geradkettiges oder verzweigtes Alkyl mit bis zu 4 Kohlenstoffatomen, Morpholin oder Phenyl bedeutet, das gegebenenfalls durch Fluor, Chlor oder Brom substituiert ist,

$R^4$ und $R^5$ gleich oder verschieden sind und Wasserstoff, Phenyl, Cyclopropyl, Cyclopentyl, Cyclohexyl oder geradkettiges oder verzweigtes Alkyl mit bis zu 5 Kohlenstoffatomen bedeuten, das gegebenenfalls durch Phenyl, Carboxy, Pyridyl oder durch geradkettiges oder verzweigtes Alkoxycarbonyl mit bis zu 4 Kohlenstoffatomen substituiert ist,

oder

$R^4$ und $R^5$ gemeinsam mit dem Stickstoffatom einen Piperazin-, Morpholin- oder Piperidinring bilden, die gegebenenfalls im Fall des Piperazinrings, gegebenenfalls auch über das Stickstoffatom, bis zu 2-fach gleich oder verschieden durch Benzyl, Formyl, Carboxy, geradkettiges oder verzweigtes Alkoxycarbonyl mit bis zu 3 Kohlenstoffatomen, Benzyloxycarbonyl oder durch einen Rest der Formel $-CH_2-CO-NR^{13}R^{14}$ substituiert ist,

worin

$R^{13}$ und $R^{14}$ gemeinsam mit dem Stickstoffatom einen Piperazin-, Piperidin- oder Pyrrolidinring bilden,

$R^6$ Phenyl oder geradkettiges oder verzweigtes Alkyl mit bis zu 4 Kohlenstoffatomen bedeutet,

$R^7$ und $R^8$ gleich oder verschieden sind und Wasserstoff, Phenyl, geradkettiges oder verzweigtes Alkyl oder Acyl mit jeweils bis zu 4 Kohlenstoffatomen oder einen Rest der Formel $-C(S)-NR^{15}R^{16}$ bedeuten,

worin

$R^{15}$ Wasserstoff oder Methyl bedeutet

und

$R^{16}$ geradkettiges oder verzweigtes Alkoxycarbonyl mit bis zu 4 Kohlenstoffatomen bedeutet,

$R^9$ und $R^{10}$ gleich oder verschieden sind und Wasserstoff, geradkettiges oder verzweigtes Alkyl mit bis zu 4 Kohlenstoffatomen, Phenyl oder einen Pyrimidin-, Pyridyl-, Thiazolyl- oder 1,2,4-Thiadiazolylring bedeuten, wobei die Cyclen gegebenenfalls durch Hydroxy, Carboxy oder durch geradkettiges oder verzweigtes Alkyl, Alkoxy oder Alkoxycarbonyl mit jeweils bis zu 4 Kohlenstoffatomen substituiert sind,

D ein Sauerstoff- oder Schwefelatom bedeutet,

$R^{11}$ und $R^{12}$ gleich oder verschieden sind und Phenyl bedeuten, das gegebenenfalls durch Fluor, Chlor oder Brom substituiert ist

und deren Salze.

**4.** Verfahren zur Herstellung der Verbindungen der allgemeinen Formel (I), gemäß Anspruch 1, dadurch gekennzeichnet, daß man

Verbindungen der allgemeinen Formel (II)

$A-NH-CO-CH_2-L$ (II)

in welcher

A die in den Ansprüchen 1 bis 3 angegebene Bedeutung hat

und

L für eine typische Abgangsgruppe, vorzugsweise für Chlor oder Brom steht,

mit 4-(2-Oxo-1-benzimidazolinyl)-piperidin oder Formel (III)

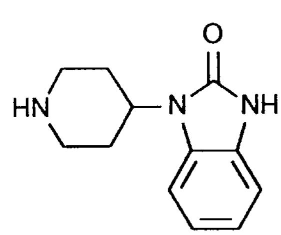

(III).

in inerten Lösemitteln, gegebenenfalls in Anwesenheit einer Base und/oder Hilfsstoffes umsetzt, und gegebenenfalls die unter A aufgeführten Substituenten nach üblichen Methoden, wie beispielsweise Alkylierung, Acyclierung, Verseifung, Hydrierung, Amidierung oder Sulfonamidierung derivatisiert.

**5.** Arzneimittel enthaltend eine oder mehrere Verbindungen aus den Ansprüchen 1 bis 3.

**6.** Verwendung der Verbindungen aus den Ansprüchen 1 bis 3 zur Herstellung von Arzneimitteln.

Europäisches Patentamt

# EUROPÄISCHER RECHERCHENBERICHT

Nummer der Anmeldung
EP 94 10 8130

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | CHEMICAL ABSTRACTS, vol. 84, no. 9, 1976, Columbus, Ohio, US; abstract no. 59466u, ISAMU MARUYAMA ET AL 'Aniline derivatives' Seite 527 ; * Zusammenfassung * & JP-A-50 108 264 (SUMITOMO CHEMICAL CO.) --- | 1 | C07D401/04<br>A61K31/445<br>C07D401/14<br>C07D417/14 |
| A | EP-A-0 092 391 (KYOWA HAKKO KOGYO) 26. Oktober 1983 * Anspruch 1 * ----- | 1 | |

RECHERCHIERTE SACHGEBIETE (Int.Cl.5)

C07D
A61K

Der vorliegende Recherchenbericht wurde für alle Patentansprüche erstellt

| Recherchenort | Abschlußdatum der Recherche | Prüfer |
|---|---|---|
| DEN HAAG | 3. August 1994 | Voyiazoglou, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)